# EUROPEAN PATENT SPECIFICATION

(11) **EP 3 645 026 B1**
(45) Date of publication and mention of the grant of the patent: **04.06.2025**
(21) Application number: 17749216.2
(22) Date of filing: 28.06.2017
(51) Int. Cl.: A61K 35/747, C12N 1/20, A61P 1/12, C12R 1/225

(54) **NEW STRAIN OF LACTOBACILLUS REUTERI**
NEUER STAMM VON LACTOBACILLUS REUTERI
NOUVELLE SOUCHE DELACTOBACILLUS REUTERI

(43) Date of publication of application: 06.05.2020
(73) Proprietor: NOOS S.r.l., 00181 Roma (RM) (IT)
(72) Inventor: ELLI, Marina, 29017 Fiorenzuola d'Arda (PC) (IT)
(74) Representative: Di Giovine, Paolo
(86) International application number: PCT/IB2017/053856
(87) International publication number: WO 2019/002914

(56) References cited:
- WO-A1-2012/050826
- SZAJEWSKA H ET AL: "Meta-analysis: Lactobacillus reuteri strain DSM 17938 (and the original strain ATCC 55730) for treating acute gastroenteritis in children", BENEFICIAL MICROBES, vol. 5, no. 3, 1 September 2014 (2014-09-01), pages 285 - 293, XP055452938, ISSN: 1876-2883
- MIGLENA GEORGIEVA ET AL: "USE OF THE PROBIOTIC Lactobacillus reuteri DSM 17938 IN THE PREVENTION OF ANTIBIOTIC-ASSOCIATED INFECTIONS IN HOSPITALIZED BULGARIAN CHILDREN: A RANDOMIZED, CONTROLLED TRIAL", JOURNAL OF IMAB - ANNUAL PROCEEDING (SCIENTIFIC PAPERS), vol. 21, no. 4, 8 October 2015 (2015-10-08), pages 895 - 900, XP055443537, DOI: 10.5272/jimab.2015214.895
- SHORNIKOVA A-V ET AL: "LACTOBACILLUS REUTERI AS A THERAPEUTIC AGENT IN ACUTE DIARRHEA IN YOUNG CHILDREN", JOURNAL OF PEDIATRIC GASTROENTEROLOGY AND NUTRITION, LIPPINCOTT WILLIAMS WILKINS, INC, US, vol. 24, no. 4, 1 April 1997 (1997-04-01), pages 399 - 404, XP001013327, ISSN: 0277-2116, DOI: 10.1097/00005176-199704000-00008
- CLEUSIX VALENTINE ET AL: "Inhibitory activity spectrum of reuterin produced by Lactobacillus reuteri against intestinal bacteria", BMC MICROBIOLOGY, BIOMED CENTRAL LTD, GB, vol. 7, no. 1, 12 November 2007 (2007-11-12), pages 101, XP021033242, ISSN: 1471-2180

## Description

The present invention relates to the strain of *Lactobacillus reuteri* LMG P-27481 provided with anti-inflammatory activity, immunomodulatory activity and capability of inhibiting pathogenic microorganisms. The microorganism can result to be useful and contribute as probiotic in improving the man health status and preventing and/or treating gastrointestinal disturbances, with particular reference to the treatment of diarrhoea caused by *Clostridium difficile.* The invention also relates to pharmaceutical compositions or food or medical devices comprising the strain of *Lactobacillus reuteri* LMG P-27481.

### State of art

Diarrhoea is defaecation disturbance characterized by an increase in the emission of a daily quantity of faeces higher than 200 g with decrease in the consistency thereof and by an increase in the frequency of discharging the intestinal bowels. It can be acute if the duration is shorter than two weeks, persistent if it is comprised between two and four weeks and chronical if it has a higher duration (1). The acute diarrhoea is triggered in 70% of cases by infective agents (such as *Staphylococcus aureus, Escherichia coli, Clostridium difficile, Salmonella* spp., Norovirus, etc.), but it can also be due to the use of drugs (ex. antibiotics, chemiotherapeutic substances), or to some pathologies (diverticulitis, intestinal ischaemia, allergies or intolerances). The acute diarrhoea due to infective cause is a serious problem in the developing countries, above all for the paediatric population, wherein, annually, at least 4 millions of children being less than 5 years old die. This pathology, in fact, can propagate easily in areas being under precarious hygienic-sanitary conditions: lack of drinking water, crowding, presence of not disposed waste, inadequate cooking of some kinds of food. Diarrhoea due to infective causes can be accompanied by other symptoms, which depend upon the type of microorganism responsible for the pathological status. One can have nausea, vomiting, fever and diarrhoea can be aqueous or sanguinolent. The microorganisms capable of invading the intestinal epithelium *(Salmonella, Shigella,* etc.) or of producing cytotoxic toxins *(Clostridium difficile)* determine abdominal pain, high fever and sanguinolent diarrhoea. As far as the treatment is concerned, since diarrhoea is expression of an underneath pathological process, the antidiarrhoeal drug can be only a palliative measure, until the organism itself or the physician finds a remedy for the underlying cause. First of all diarrhoea alters the hydro-electrolytic equilibrium of the organism, therefore, even before administering antidiarrhoeal drugs, it is necessary to provide for the reintegration of liquids, salts and sugars. To this purpose on the market there are specific oral rehydration solutions, containing electrolytes and glucose, the use thereof is recommended both by WHO (World Health Organization) and by the International paediatric companies (ESPGHAN, ESPID) (2). In most serious cases, one can have recourse to the use of electrolytic solutions by intravenous route. In case of acute diarrhoea due to bacterial infection the therapy by means of antibiotics is controversial, as often these infections tend to autolimit and the diarrhoea itself contributes in reducing the intestinal microbial count.

On the contrary, an alternative can be the probiotics, the use thereof has revealed to be effective in preventing and treating most diarrhoea typologies (Rotavirus acute diarrhoea and infective diarrhoea, diarrhoea associated to the ingestion of antibiotics, *Clostridium difficile* diarrhoea, radiation therapy diarrhoea, traveller's diarrhoea, enteral nutrition diarrhoea, etc.), through the competitive inhibition of the pathogens, the stabilization of the residing microbiota and the mitigation of the increase in the intestinal permeability for example associated to the Rotavirus infections.

However, according to the most recent meta-analyses, the effectiveness of the probiotics would not result to be confirmed by clinical data in case of some diarrhoeae, such as the traveller's diarrhoea (3). Moreover, nowadays, the greatest records about the effectiveness of probiotics substantially are restricted to the paediatric patients, wherein the beneficial action of the probiotics would express mainly through the reduction in the duration of diarrhoea and fever states, but not in the number of daily evacuations and side symptoms, for example vomiting. In adults, instead, the beneficial action of the probiotics has not been entirely demonstrated, even if one can assume that the results obtained in children can be extended even to adult population. It is further to be underlined that not all probiotics resulted to be effective, and possible advantages are absolutely strain-specific, apart from the fact that most available studies relate to Rotavirus diarrhoea (3).

The probiotics, also commonly called lactic ferments, can be defined as live microorganisms which, once ingested in adequate quantities, exert advantageous effects on the health of whoever ingests them (4-6). The lactic ferments include indeed microorganisms and, precisely, live and vital lactic bacteria, that is capable of reproducing (5-7).

The strains of bacteria included in the different preparations of ferments are several (8). The micro organisms included in the lactic ferments, in order to be effective and well tolerated during treatment, first of all must have particular features and precisely (4,5):
- they must not be pathogen and they must not cause infections or other pathologies to whoever ingests them
- they must be capable of resisting to the acidity of the gastric acid and to the bile
- they must be capable of colonizing, at least temporarily, the intestine
- they must be capable of producing antimicrobic substances (that is active against other microorganisms dangerous for our organism).

The lactic ferments do not act only by restoring the equilibrium of the intestinal microflora (9), as it was believed some time ago, but it turned out that, among the main action mechanisms, they can determine an effect:
- of protective type, above all against the infections on the intestinal mucosa (4,5)
- of positive modulation of the immune system (4,5)
- of inhibition of adhesion on the intestinal wall and then of the growth in the pathogenic bacteria (5)

From the clinical point of view, then, the lactic ferments were studied above all in the prevention and treatment of different intestinal pathologies (8). In gastroenteritis, for example, the administration of lactic ferments allowed to reach a reduction in symptomatology in shorter time (10). In the diarrhoeae of infective origin the administration of lactic ferments demonstrated to be effective in reducing both the duration and the seriousness of diarrhoea (11). In diarrhoeae due to the ingestion of antibiotics (a situation affecting 1 subject every 5 subjects ingesting antibiotics), wherein the antibiotics themselves determine an alteration of the intestinal microflora, the administration of some lactic ferments containing adequate quantities of bacteria decreased both the risk of developing diarrhoea during the antibiotic treatment, and the duration and the seriousness of diarrhoea once the same has appeared (10). In the Irritable Bowel Syndrome (IBS) the ingestion of lactic ferments demonstrated a significant reduction in pain and abdominal swelling (8).

In the traveller's diarrhoea the preventive administration of probiotics determined a quite good protection against the intestine colonization by bacteria which could have caused intestinal infections (9).

In the chronical intestinal inflammatory disease (Crohn disease and ulcerative colitis) the use of the lactic ferments gave some positive results above all in terms of keeping the remission of symptoms (12).

Furthermore, there are several patents generally relating to the use of the probiotics, alone or in association with prebiotics, in preventing or treating intestinal pathologies, with particular reference to the diarrhoeal pathology (US20110014167A1; US005837238A; WO2002070670A1; EP2753687A1) (13-16).

In particular, the patent US20110014167A1 relates to a combination of a protobiotic *(Lactobacillus rhamnosus),* a prebiotic (inulin) and fibers (soyabean polysaccharides) for treating diarrhoea, in particular acute diarrhoea. The document claims the combination of beneficial effects of the several components of the composition on the decrease in the diarrhoeal effects, the stimulation of the immune system, the improvement of the intestinal microflora and the anti-pathogenic effects. The limits of such invention are the fact that preferably it is a liquid formulation, aimed at the nutritional needs of infants being less than 3 years old and more preferably less than 12 months old. Therefore, a rehydration solution specific for preterm infants, infants born at term or infants in the period of weaning to solid food, who for various reasons can be subjected to an alteration in the not yet well formed intestinal microflora and consequently to the appearance of diarrhoeal forms of various origin, not necessarily linked to the presence of pathogens, such as Rotavirus, *Salmonella, C. difficile* or the use of antibiotics.

The patent US005837238A instead relates to the use of one or more strains of *Lactobacillus reuteri* in the treatment of diarrhoea, in particular diarrhoea caused by Rotavirus. The invention provides the administration of the bacterial cells lyophilised in a liquid suspension (water, fruit juice, milk, yogurt, etc.) or alternatively in gelatine capsules, at the concentration of at least 10⁸ cells per day, for a period of one to seven days, based upon the seriousness of the gastroenteritis, to decrease diarrhoea and vomiting. To support such indication a double-blind clinical study is mentioned, wherein the strain *L. reuteri* SD2112 led to a decrease in the duration of aqueous diarrhoea and vomiting in children being from 6 and 36 months old. 75% of the treated patients was affected by Rotavirus. No evidence was shown to support possible positive effects of the invention on other intestinal pathogens, such as *Shigella, Salmonella, S. aureus* or *C. difficile.*

The patent WO2002070670A1 relates to the strain *Lactobacillus reuteri* Probio-16 and to its features of *in vitro* inhibition of Rotavirus and other pathogenic organisms. The subject bacterium was isolated from pigs, then it has not human origin, and the patent does not show any study related to the ingestion thereof, for short or long periods, by man, and this constitutes a great limitation in terms of use safety for a probiotic.

The patent EP2753687A1 relates to the use of one or more bacterial strains, belonging to the genus of *Lactobacillus* and/or *Bifidobacteria,* for inhibiting or decreasing the growth of various biotypes of *E. coli* and of clostridia, including C. *difficile, Listeria monocytogenes, Enterococcus sp.* and *Klebsiella sp.* However, such document shows only *in vitro* evidence of the activity inhibiting the several studied bacteria and it does not mention also studies in animals or man which, on one side, could confirm the *in vivo* effectiveness, on the other side be evidence of the use safety profile.

Szajewska H et al "Meta-analysis: Lactobacillus reuteri strain DSM 17938 (and the original strain ATCC 55730) for treating acute gastroenteritis in children", and Miglena Georgieva et al "USE OF THE PROBIOTIC Lactobacillus reuteri DSM 17938 IN THE PREVENTION OF ANTIBIOTIC-ASSOCIATED INFECTIONS IN HOSPITALIZED BULGARIAN CHILDREN: A RANDOMIZED, CONTROLLED TRIAL" disclose the use of a *L. reuteri* strain (DSM 17938) for treating diarrhoea induced by C. *difficile.*

All previous documents did not succeed in solving completely the problem of treating diarrhoeal disturbances, in particular caused by *Clostridium difficile* infections, or since they generally treated the diarrhoeal problem as a symptom of an underneath conditions, or since they did not show direct proof of the treatment effect on *C*. *difficile.* Now it has been surprisingly found that the strain *Lactobacillus reuteri* LMG P-27481 succeeds in overcoming the problems of the state of art as it has optimum probiotic properties, in terms of colonization, resistance to gastric acids and to biliary salts, safety profile and immune system stimulation. Furthermore, this strain demonstrated important anti-microbic capability against the most common intestinal pathogens (ex. E. *coli, Salmonella,* Rotavirus) and more in particular against the pathogen *Clostridium difficile,* the growth thereof is strongly reduced, both in *in vitro* tests and in experiments on animals. For these reasons the strain *Lactobacillus reuteri* LMG P-27481 offers as optimum candidate in preventing and treating the diarrhoeal pathology, especially the one induced by *C*. *difficile.*

Lactobacilli are microorganisms widely present in nature, they metabolize anaerobically the carbohydrates to produce lactic acid. They are used very much for their probiotic features and they result to be useful for the intestinal flora equilibrium.

*Lactobacillus reuteri* is a bacterial species belonging to the genus *Lactobacillus,* phylum *Firmicutes,* represented in the human and animal gastroenteric tract, with optimum growth at 37°C and obliged heterofermentative profile, leading to the formation of carbon dioxide, ethanol, acetic acid, lactic acid, after fermentation of sugars. Moreover, *L. reuteri* produces anti-microbial substances, as well as folate and cobalamin (vitamin B12).

The invention is set out in the appended set of claims.

The present invention relates to:
1. the strain of *Lactobacillus reuteri* LMG P-27481
2. the strain of *Lactobacillus reuteri* LMG P-27481 for use in the treatment of diarrhoeal infections induced by *Clostridium difficile*
3. pharmaceutical compositions for the treatment of diarrhoeal infections induced *by Clostridium difficile*
4. food supplements and medical devices useful in the treatment of diarrhoeal infections induced by *Clostridium difficile*

### Brief description of the figures

The following figures are enclosed to the present description:
- Figure 1: *In vitro* survival of the strain *L. reuteri* LMG P-27481 to the gastric transit
- Figure 2: Resistance of the strain *L. reuteri* LMG P-27481 to bile salts
- Figure 3: Analysis of the cells and supernatants for expressing the markers of Interleukin 10
- Figure 4: Analysis of the cells and supernatants for expressing the markers of Interleukin 12
- Figure 5: Determination of the anti-inflammatory index of *L. reuteri* LMG P-27481 and of the reference strains
- Figure 6: Body weight of the animals in the CD spontaneous infection after antibiotic treatment. The comparison of the body weight of mice between beginning of treatment (white column) and end of treatment (black column) is shown. Group 1 (check) did not receive any treatment. Group 2 received only antibiotic for 2 weeks. Group 3 received the antibiotic therapy for 2 weeks and concomitant treatment with probiotic. (*) supplied statistically significant with respect to the same animals at the beginning of treatment.
- Figure 7: Levels of mucosal inflammation, expressed in terms of activity of myeloperoxidase (MPO), in the mucous membrane of the colon of animals developing CD spontaneous infection after antibiotic therapy. Group 1 (check) did not receive any treatment. Group 2 received only antibiotic for 2 weeks. Group 3 received the antibiotic therapy for 2 weeks and concomitant treatment with probiotic. (*) supplied statistically significant with respect to the group treated with antibiotic for 2 weeks (group 2).
- Figure 8: Body weight of the animals in the infection induced by CD after antibiotic treatment. Group 1 (check) did not receive any treatment. Group 2 received antibiotic and challenge with CD. Group 3 received the antibiotic therapy, CD and *L. reuteri* LMG P-27481 before the end of the antibiotic therapy. Group 4 received the antibiotic therapy, CD and L. *reuteri* LMG P-27481 after the challenge with CD. (*) supplied statistically significant with respect to the same animals at the beginning of the treatment.
- Figure 9: Detection of CD DNA by means of RT-PCR in the samples of faeces of the different experimental groups. Group 2 received antibiotic and challenge with CD. Group 3 received the antibiotic therapy, CD and L. *reuteri* LMG P-27481 before the end of the antibiotic therapy. Group 4 received the antibiotic therapy, CD and *L. reuteri* LMG P-27481 after the challenge with CD. (*) supplied statistically significant with respect to group 2.
- Figure 10: Levels of mucosal inflammation, expressed in terms of MPO activity, in the intestinal mucous membrane of animals with infection induced by CD after antibiotic therapy. Group 1 (check) did not receive any treatment. Group 2 received antibiotic and challenge with CD. Group 3 received the antibiotic therapy, CD and *L. reuteri* LMG P-27481 before the end of the antibiotic therapy. Group 4 received the antibiotic therapy, CD and L. *reuteri* LMG P-27481 after the challenge with CD. (*) supplied statistically significant with respect to group 2.

### Description of the invention

The strain, the present invention relates to, is *Lactobacillus reuteri* LMG P-27481 filed on 1 March 2013 at the center for the collection of microorganisms BCCM/LMG Bacteria Collection of Gent University (Belgium), authorized pursuant to the Budapest Treaty) by Moviscom srl (Via dei Pirenei 10 00144 Rome Italy) and subsequently - on 13 February 2015 - assigned definitively to Probioresearch srl including the right of making reference to the biological material filed in the patent application and the unconditional and irrevocable consent that the filed material is made available to the public according to Rule 33 EPC.

Such strain was studied in details for its genetic characterization and for its functional features in order to identify its probiotic activity by means of:
- Isolation and purification
- Taxonomic identification
- Biosafety
- *In vitro* functional evaluations

### Isolation and purification

The strain isolation was performed by screening nr. 21 faecal samples of as many healthy children being between 6 months and 4,5 years old. Such samples were obtained with the informed consent of parents of children.

### Taxonomic identification

The strain was subjected to taxonomic identification by means of genus-, species- and strain-specific identification performed with PCR technique.

The sequencing of the codifying gene for 16SrRNA was further performed by means of DNA amplification and subsequent display and quantification on agarose gel. The sequencing was checked with respect to a positive check of another known strain of *Lactobacillus reuteri.*

As to ***Biosafety*** of the strain *L. reuteri* LMG P-27184:
- sensitivity to antibiotics
- plasmids' profile
were studied

### Sensitivity to antibiotics

The minimum inhibiting concentrations (MICs) of 15 antibiotics were determined by using ISO 10932:2010 method as suggested in the Scientific Opinion EFSA 2012 (17). As inner checking of the effectiveness and reliability of the performed tests the strain *Lactobacillus paracasei* ATCC 334 was taken pursuant to the above-mentioned ISO rule.

The strain *Lactobacillus reuteri* LMG P-27481 resulted to be sensitive to all antibiotics considered by EFSA in defining the cuts-off for the species *L. reuteri.* The strain, then, is provided with the fundamental requirement of sensitivity to antibiotics required to guarantee the use safety in man.

### Plasmids' profile

The presence of plasmids was verified to check the potential effect of genetic transmission of resistance to antibiotics. The strain was subjected to the extraction of plasmids by means of the alkaline lysis by Anderson & McKay 1983 (18) with the purpose of verifying the presence of mobile genetic elements inside the strain cells. For the strain *Lactobacillus reuteri* LMG P-27481 the absence of plasmids was noted. This piece of data is particular important as it does not allow the genetic transmission of resistance to antibiotics by the tested strain.

For the ***in vitro* functional characterization** of the strain the following parameters were determined:
- Tolerance to the gastric transit
- Tolerance to the intestinal transit
- Production of hydrogen peroxide

### Tolerance to the gastric transit

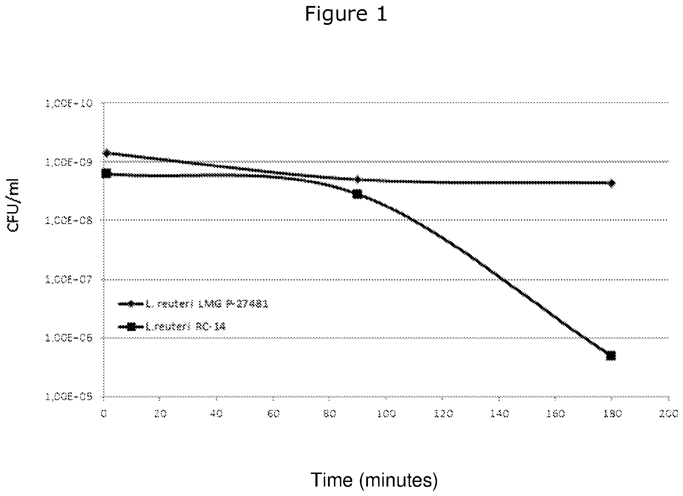

The analysis of resistance of the strain to the simulated gastric acid was performed according to the indications provided by Charteris et al. 1998 (19), by using as reference strain the *Lactobacillus reuteri* RC-14. A significant capability of the strain LMG P-27481 of surviving to the simulated gastric transit was noted, contrary to what found for the reference strain. After 3 hours of contact with the simulated gastric acid the vitality of *L. reuteri* LMG P-27481 resulted to be still optimum, whereas for the reference strain a high loss in vitality was noted (corresponding to 2.76 log10 UFC/ml) (Fig. 1).

### Tolerance to the intestinal transit

The capability of the strain to resist to the conditions of intestinal transit by means of the exposition to the simulated pancreatic juice and bile salts was determined, by means of *in vitro* tests. As reference, the strain of *Lactobacillus reuteri* RC-14 was used.

### ➢ Tolerance to the simulated pancreatic juice

The tolerance to the simulated pancreatic juice was performed according to the indications provided by Charteris et al. 1998 (19).

*Lactobacillus reuteri* LMG P-27481 showed a strong resistance to the exposure to the simulated pancreatic juice up to 4 hours of co-incubation. Even the reference strain revealed a good tolerance, even if to lesser extent than L. *reuteri* LMG P-27481 (Table 1).

**Table 1**

| **Resistance to the simulated pancreatic juice** | | |
|---|---|---|
| STRAIN | T1 (1 min. contact) | T240 (240 min. contact) |
| *L. reuteri* LMG P-27481 | 1.06 x10⁹ UFC/ml | 1.49 x10⁹ UFC/ml |
| *L. reuteri* RC-14 | 8.09 x10⁸ UFC/ml | 7.73 x10⁸ UFC/ml |

### ➢ The tolerance to bile Salts

The analysis was performed by comparing the capability of the strain *L. reuteri* LMG P-27481 with respect to *L. reuteri* RC-14 in growing in selective medium for lactobacills with and without the addition of 0.3% of lyophilised bile Salts. The microorganisms were incubated at 37°C for about 6 hours in microaerophily by monitoring the growth each hour during the incubation period. The reference method is that of Gilliland et al. 1984 (20).

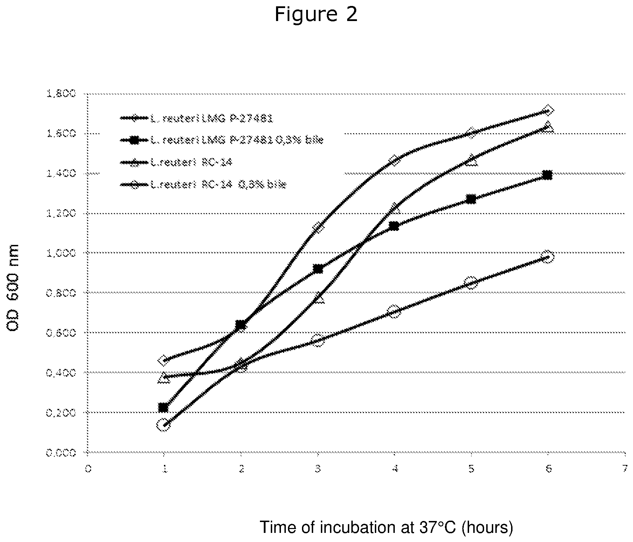

The dynamics of sensitivity of the strains to the bile presence in the culture medium is represented in figure 2. *Lactobacillus reuteri* LMG P-27481 shows a good tolerance to bile, whereas the reference strain reveals a greater susceptibility with marked reduction in the Optical Density with respect to its own checking grown on not additioned normal medium.

### Production of hydrogen peroxide

The production of hydrogen peroxide by the strain was determined by quantitative route based upon the method of Yap & Gilliland 2000 (21). The results, shown in table 2, show the levels of H₂O₂ produced after 1 and 24 hours of incubation at 5°C under conditions of microaerophily.

**Table 2**

| **Production of hydrogen peroxide by the tested strain** (µg/ml/10⁹ CFU) | | |
|---|---|---|
| Strain | H₂O₂ (µg/ml/10⁹ CFU) 1 hour | H₂O₂ (µg/ml/10⁹ CFU) 24 hours |
| *L. reuteri* LMG P-27481 | 2.58 | 3.96 |

The strain *L. reuteri* LMG P-27481, characterized as described above, demonstrated to have probiotic activity and it was further studied to check if it would be suitable in preventing/treating diarrhoea of various origin.

Within the characterization of the effectiveness of this strain in treating and/or preventing diarrhoea, other reference probiotic strains were taken into consideration with the purpose of verifying and quantifying the effect of the strain under study. In particular the bacterial strains used as reference were:
- *Lactobacillus reuteri* DSM 17938;
- *Lactobacillus rhamnosus* ATCC 53103 (also known as LGG^{®})

One chose to use exactly these two strains as reference, since there is wide literature related to the their effects in the treatment of diarrhoea, in particular acute diarrhoea. The invention also relates to
- pharmaceutical compositions comprising the strain, thereto the invention relates, and pharmaceutically tolerable additives and use thereof in the treatment of diarrhoeal infections due to *Clostridium difficile.*
- medical devices comprising the strain as claimed in claims 1 to 3 and additives usually used in the field for use in the treatment of diarrhoeal infections induced by *Clostridium difficile*
- food supplements comprising the strain of the invention and nutraceutically tolerable additives useful in the treatment of diarrhoeal infections induced by *Clostridium difficile.*

### Section VIII: Examples

### Example 1

### Inhibiting activity of L. reuteri LMG P-27481 against intestinal pathogenic bacteria

Aim: checking the capability of the trial strains to exert an inhibiting action against intestinal pathogens.

The pathogens used in the assays were obtained by TCC and by Culture Collection, University of Göteborg (Sweden). The bacterial strains were kept on Rogosa agar (L. *reuteri* and *L. rhamnosus),* LB *(E. coli, S. aureus, Salmonella)* and BHI (C. *difficile*). Rotavirus (ATCC VR-2018) was extended and titrated on cells MA-104 (rhesus monkey kidney).

The two strains of *L. reuteri* and *L. rhamnosus* were inoculated separately at concentrations of 10⁷ CFU/ml and grown in broth MRS for 24 hours in anaerobiosis. At the end of incubation the culture was centrifuged (13.000 g at 4°C), the supernatant was filtered (0.22 µm) and pH was corrected at 7.0. Then, this medium was inoculated with *E. coli, S. aureus, Salmonella* or C. *difficile* (10⁷ CFU/ml) and incubated at 37°C. An aliquot of the medium was collected at different time intervals (6, 12, 24 hours from inoculum) and the live bacteria quantified by means of vital microbical count with serial dilutions sown on suitable agarized medium plates, incubated at 37°C in aerobiosis (E. *coli, S. aureus, Salmonella)* or anaerobiosis (*C*. *difficile).*

For each assay 3 experiments were performed with triplicate determinations.

**Table 3**

| **Quantitative response of *Escherichia coli* upon contact with the tested strains (CFU/ml)** | | | |
|---|---|---|---|
| | ***E. coli 6 h*** | ***E. coli 12 h*** | ***E. coli 24 h*** |
| ***Check*** | 2.72±0,4 x 10⁸ | 3.64±0.3 x 10⁸ | 3.87±0.3 x 10⁸ |
| ***L. reuteri LMG P-*27481** | 1.29±0.3 x 10⁸ | 2.16±0.6 x 10⁸ | 1.96±0.3 x 10⁸ |
| ***L. reuteri DSM* 17938** | 2.68±0.2 x 10⁸ | 3.02±0.4 x 10⁸ | 1.75±0.2 x 10⁸ |
| ***L. rhamnosus ATCC 53103*** | 2.43±0.4 x 10⁸ | 3.52±0.6 x 10⁸ | 3,83±0.7 x 10⁸ |

**Table 4**

| **Quantitative response of *Salmonella* upon contact with the tested strains (CFU/ml)** | | | |
|---|---|---|---|
| | ***Salmonella 6 h*** | ***Salmonella 12 h*** | ***Salmonella 24 h*** |
| ***Check*** | 4.05±0.2 x 10⁸ | 4.54±0.2 x 10⁸ | 5.99±0.4 x 10⁸ |
| ***L. reuteri LMG P-27481*** | 3.23±0.5 x 10⁸ | 3.06±0.2 x 10⁸ | 3.73±0.2 x 10⁸ |
| ***L. reuteri DSM* 17938** | 2.59±0.4 x 10⁸ | 2.69±0.4 x 10⁸ | 2.85±0.3 x 10⁸ |
| ***L. rhamnosus ATCC 53103*** | 3.44±0.5 x 10⁸ | 3.,35±0.3 x 10⁸ | 3.94±0.3 x 10⁸ |

**Table 5**

| **Quantitative response of *Staphylococcus aureus* upon contact with the tested strains (CFU/ml)** | | | |
|---|---|---|---|
| | ***S*. *aureus* 6 *h*** | ***S*. *aureus* 12 *h*** | ***S*. *aureus* 24 *h*** |
| ***Check*** | 3.70±0.2 x 10⁸ | 4.03±0.3 x 10⁸ | 4.36±0.6 x 10⁸ |
| ***L. reuteri LMG P-27481*** | 3.66±0.2 x 10⁸ | 3.72±0.4 x 10⁸ | 3.93±0.2 x 10⁸ |
| ***L. reuteri DSM* 17938** | 3.53±0.3 x 10⁸ | 3.49±0.4 x 10⁸ | 3.56±0.4 x 10⁸ |
| ***L. rhamnosus ATCC 53103*** | 3.48±0.5 x 10⁸ | 3.35±0.6 x 10⁸ | 4.20±0.5 x 10⁸ |

**Table 6**

| **Quantitative response of *Clostridium difficile* upon contact with the tested strains (CFU/ml)** | | | |
|---|---|---|---|
| | ***C. difficile 6 h*** | ***C. difficile 12 h*** | ***C. difficile 24 h*** |
| ***Check*** | 61.6±3.1 x 10⁶ | 81.3±11.3 x 10⁶ | 13.0±4.7 x 10⁷ |
| ***L. reuteri LMG P-27481*** | 46.3±3.5 x 10⁶ | 72.1 ±3.1 x 10⁶ | 46.3±3.5 x 10⁶ |
| ***L. reuteri DSM 17938*** | 50.7±5.1 x 10⁶ | 71.4±4.5 x 10⁶ | 10.9±8.5 x 10⁷ |
| ***L. rhamnosus ATCC 53103*** | 36.3±4.6 x 10⁶ | 69.7±4.2 x 10⁶ | 11.0±6.2 x 10⁷ |

From the results it is noted that both strains of *L. reuteri* reduce the growth of the Gram negative intestinal pathogens *E. coli* and *Salmonella,* in similar extent therebetween. *Lactobacillus rhamnosus* ATCC 53103 does not show any inhibiting activity against *E*. *coli* and a clearly lower action towards *Salmonella.* As far as *S*. *aureus* is concerned, a minimum inhibiting activity for the 2 strains of *L. reuteri* is noted, whereas *L. rhamnosus* does not show any activity even towards this microorganism (Tables 3, 4 and 5).

A separate discussion should be made for *C*. *difficile,* therefor only the strain *L. reuteri* LMG P-27481 showed to be capable of reducing significantly the growth thereof (Table 6).

### Example 2

### Inhibiting activity of L. reuteri LMG P-27481 against Rotavirus

The aim of these experiments was to verify the capability of trial strains to protect human intestinal epithelial cells from Rotavirus infection.

An *in vitro* model with human intestinal epithelial cells (HT-29) sensitive to the natural infection with human Rotavirus was used. The capability of the strain *L. reuteri* LMG P-27481 was checked, compared to another strain of *L. reuteri* (DSM 17938) and to *Lactobacillus rhamnosus* ATCC 53103, to influence the Rotavirus infection of HT-29 cells, by using two experimental designs:
**A) Pre-treatment** - Confluent monolayers of HT-29 cells were washed with DMEM without antibiotic and then incubated with DMEM medium containing the strains under examinations at a multiplicity of infection of 1:50 (epithelial cells:bacterium). The bacteria were left in contact with the epithelial cells for two hours, then the medium was removed and the infected cells for 1 hour at 37°C with Rotavirus (previously subjected to activation for 1 hour at 37°C with trypsin 10 µg/ml). After one hour the medium was removed and replaced with complete medium additioned with trypsin (2 µg/ml), necessary to guarantee the reinfection of the released viral particles. The cells were then incubated for 72 hours at 37°C. At the end of the incubation the cells and the medium were collected and one proceeded with the extraction of the total DNA and with the quantification of the copies of viral genome by means of quantitative PCR.
**B) Competition** - Confluent monolayers of HT-29 cells were washed and incubated with DMEM medium without antibiotic. Parallelly the Rotavirus was subjected to proteolytic activation (1 hour at 37°C with trypsin 10 µg/ml) and then incubated with the strains under examination (collected from a culture during the logarithmic growth and diluted corresponding to a multiplicity of infection of 1:50 on the epithelial cells). The bacteria were left in contact with the virus for 90 min at 37°C. Then the virus+bacteria mixture was centrifuged at 3500 revolutions for 10 minutes. The supernatant (without bacteria) was added to the epithelial cells for 1 hour at 37°C. After one hour the medium of the epithelial cells was removed and replaced with complete medium additioned with trypsin (2 µg/ml). The cells then were incubated for 72 hours at 37°C. At the end of incubation the cells and the medium were collected and one proceeded with the total DNA extraction. The quantification of the copies of viral genome was performed by quantitative PCR.
For each assay 3 experiments were performed with duplicate determinations.

All tested strains showed a significant capability of reducing the replication of Rotavirus in human intestinal epithelial cells in both used treatment schedules (Table 7).

**Table 7**

| ***Number of copies of viral genome depending upon the presence of the tested strains*** | | |
|---|---|---|
| | ***Pre-treatment Protocol (nr.* of *copies of the viral genome)*** | ***Competition Protocol (nr.* of *copies of the viral genome)*** |
| ***Check Rotavirus*** | 4371±660 x 10³ | 4630±720 x 10³ |
| ***L. reuteri LMG P-27481* + *Rotavirus*** | 1528±390 x 10³ | 1012±410 x 10³ |
| ***L. reuteri DSM 17938* + *Rotavirus*** | 2198±450 x 10³ | 531±85 x 10³ |
| ***L. rhamnosus ATCC 53103* + *Rotavirus*** | 627±90 x 10³ | 421±83 x 10³ |

### Example 3

### Capability of L. reuteri LMG P-27481 to adhere to a monolayer of intestinal epithelial cells

The aim of these experiments was to verify the capability of trial strains to adhere to a monolayer of human intestinal epithelial cells.

Human intestinal epithelial cells (HT29) were made to grow until forming confluent monolayers.

Forty-eight hours after having obtained confluence, the monolayers were washed and incubated with DMEM medium additioned with the strains under examination at a multiplicity of infection of 1:10 (epithelial cells: bacterium). The incubation was performed for 90 minutes at 37°C in presence of a slight oscillation of the plate to favour contact with the cells. At the end of incubation, the culture medium was eliminated, the cells washed with sterile DMEM to remove not adhering bacteria and the adhering bacterial cells were quantified by means of vital microbial count by performing serial dilutions sown on agarized MRS medium plates incubated at 37°C in anaerobiosis.

For each assay 3 experiments were performed with duplicate determinations.

All tested strains showed the capability of adhering to the human intestinal cellular monolayer. *L. reuteri* LMG P-27481 resulted to be more effective than *L. reuteri* DSM 17938 (about 1.55 times better) and similar to *L. rhamnosus* ATCC 53103 (Table 8).

**Table 8**

| ***Adhesion of the strains with human intestinal cellular line HT29*** | | |
|---|---|---|
| | **Adhesion (n. of bacteria - CFU)** | **Adhesion (%of bacteria adhering to the monolayer of epithelial cells)** |
| ***L. reuteri LMG P-27481*** | 703±23 x 10³ | 23.4% |
| ***L. reuteri DSM 17938*** | 451±54 x 10³ | 15% |
| ***L. rhamnosus ATCC 53103*** | 788±40 x 10³ | 26.3% |

### Example 4

### Immuno-modulating activity of L. reuteri LMG P-27481

The aim of these experiments was to check the immuno-modulating capability of the trial strains on human dendritic cells.

Human dendritic cells were produced starting from monocytes obtained from peripheral blood of healthy donors. The monocytes were differentiated in dendritic cells by adding granulocyte macrophage colony stimulating factor (GM-CSF, 5 ng/mL) and IL-4 (2,5 ng/mL) to obtain immature dendritic cells (DC). On the day of experiment the DCs were collected in wells, washed by means of centrifugation, counted and aliquoted to obtain the following experimental groups:
- DC
- DC + each one of the three strains under analysis
- DC + supernatant of the three strains under analysis
- DC + *Salmonella*
- DC + each one of the three strains under analysis + *Salmonella*
- DC + supernatant of the three strains under analysis + *Salmonella*

DCs were incubated with *Salmonella* (MOI 10:1 bacteria: DC) or with the strains of lactobacilli under examination (MOI 10:1) or with addition of 2% of MRS medium conditioned by probiotic strains (see above - corresponding to a MOI 10:1) for 1 hour. The cells were then washed by means of centrifugation and incubated for additional 24 hours in medium containing gentamicin (100 µg/ml). The culture supernatant was collected and used for quantifying cytokines (IL-10 and IL12p70) and subsequent determination of the anti-inflammatory index (IL- 10/IL12p70 concentration ratio).

For each assay 3 experiments were performed with duplicate determinations.

### Results

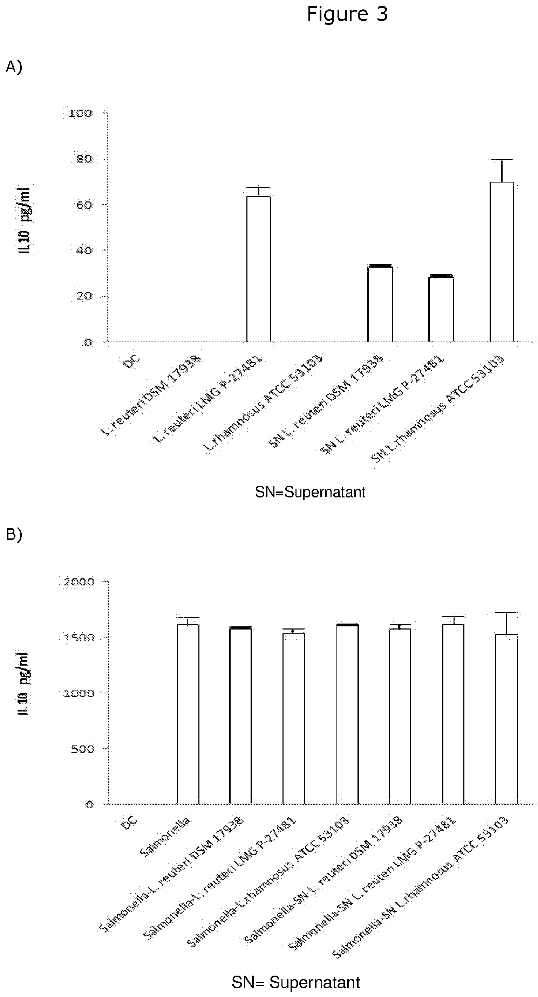

The obtained results are represented in Figures 3, 4 and 5.

It was found that the live cells of *L. reuteri* LMG P-27481, differently from *L. reuteri* DSM 17938 and *L. rhamnosus* ATCC 53103, strongly stimulate the secretion of IL-10, whereas the supernatant of all strains stimulates the secretion of IL-10 with stronger activity of *L. rhamnosus* ATCC 53103 (Figure 3A). *Salmonella* induces a significant release of IL-10 but the intervention of the probiotic strains does not produce any contribution (Figure 3B).

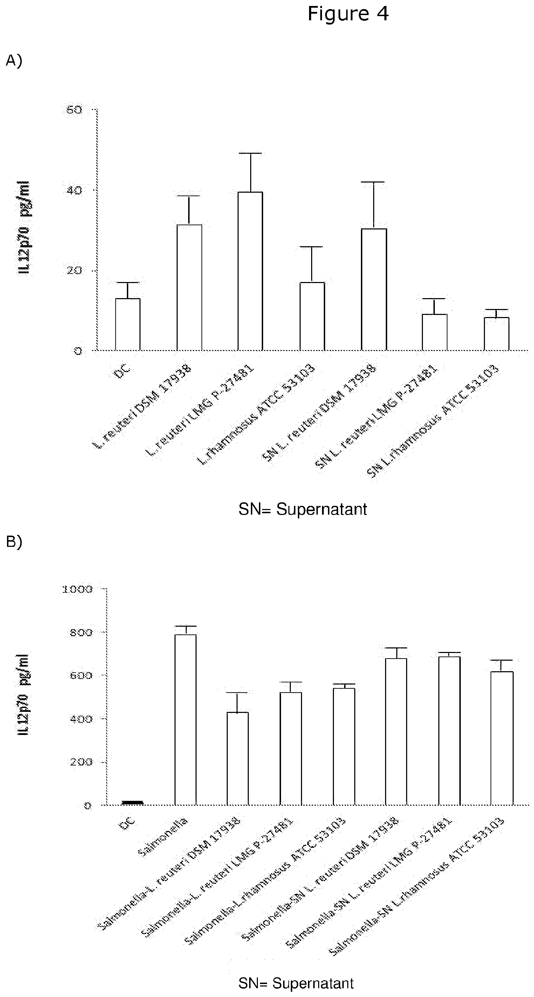

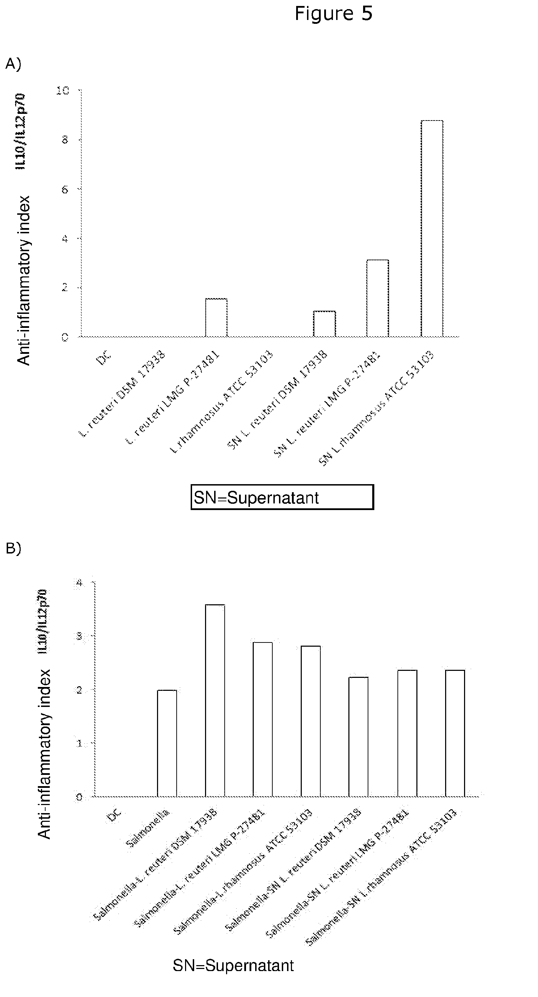

*L. reuteri* LMG P-27481 also revealed to be the most effective stimulus for the secretion of IL-12p70 whereas the supernatant shows a stimulation of IL-12 comparable for *L. reuteri* LMG P-27481 and *L. rhamnosus* ATCC 53103, lower than the one produced by *L. reuteri* DSM 17938 (Figure 4A). *Salmonella* strongly induced IL-12p70 and all strains revealed to be capable of counterbalance this effect (Figure 4B). The anti-inflammatory index (IL-10/IL-12 ratio) revealed that *L. reuteri* LMG P-27481 is the most effective strain in inducing the anti-inflammatory response (Figure 5A), whereas, in presence of *Salmonella,* all tested strains behave analogously (Figure 5B).

### Example 5

### Effect of Lactobacillus reuteri LMG P-27481 on the inflammation induced by C. difficile spontaneous infection after antibiotic treatment.

The aim of this study was to check the anti-inflammatory effect of *L. reuteri* LMG P-27481 evaluated in a murine model of C. *difficile* (CD) infection, based upon the spontaneous colonization by CD in mice subjected to wide-spectrum antibiotic treatment.

In this experimental model 6-week-old C57BI/6 mice were subjected to treatment with wide-spectrum antibiotic (cefoperazone 0.5 mg/ml) for 2 weeks. The animals received a daily supplementation of *L. reuteri* LMG P-27481 (10⁹ CFU/die) or only carrier by intragastric route in the period concomitant to the antibiotic therapy. The animals were sacrificed at the end of the 2 weeks of antibiotic therapy and concomitant administration of probiotic.

The variation in the body weight of the animals during experimentation was determined to evaluate the general state of health of the animals.

Then the study of the anti-inflammatory effect of the probiotic was performed by determining the myeloperoxidase (MPO) in the colon wall and the histological analysis of cecum sections.

### Body weight

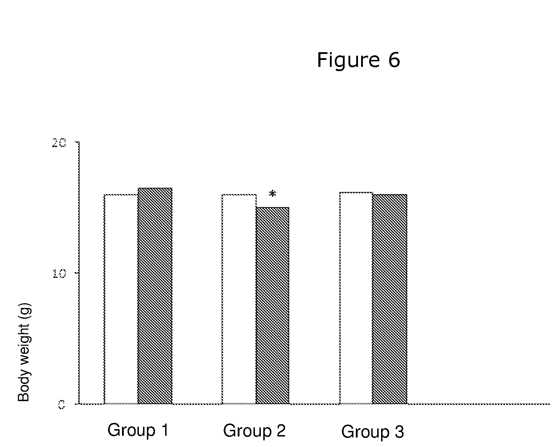

The checking mice not treated with antibiotic showed a relative stability of the body weight whereas the mice subjected for 2 weeks to antibiotic therapy showed a significant weight reduction. The administration of the probiotic *L. reuteri* LMG P-27481 contrasted the weight loss due to the antibiotic and the animals showed a trend similar to the checks which did not received antibiotic (Figure 6).

### Myeloperoxidase (MPO)

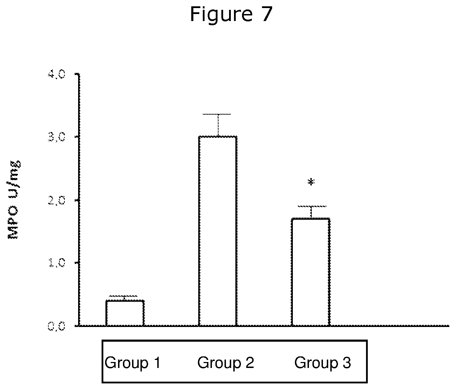

The inflammation at the level of the intestinal mucous membrane, in terms of infiltration due to neutrophils, was quantified by measuring the level of activity of MPO in the colon wall. As shown in Figure 7, the checking mice (without any treatment) showed a very low level of MPO activity, differently from the mice subjected to antibiotic treatment, which showed a consistent increase in the MPO level.

The administration of the probiotic *L. reuteri* LMG P-27481 concomitantly with the antibiotic treatment determined a significant reduction in the MPO activity.

### Histopathology of intestinal mucous membrane

The extension of inflammation at mucosal level was also evaluated by histopathological analysis. It was found that in the caecum of the mice which received the antibiotic administration for 2 weeks there is a condition of mucosal and submucosal inflammation associated to a slight epithelial damage. In the mice which ingested L. *reuteri* LMG P-27481 during the antibiotic therapy the extension of mucosal inflammation results to be reduced, as well as the submucosal oedema.

The probiotic, ingested contemporary to the antibiotic results to be effective in reducing the seriousness of the effects associated to the antibiotic therapy. The spontaneous reversion of the negative effects associated to the antibiotic appears both with reference to the body weight which tends spontaneously tends to return to normality, and in relation to the inflammation level of the intestinal mucous membrane.

### Example 6

### Effect of Lactobacillus reuteri LMG P-27481 in a model of challenge with C. difficile (CD) after antibiotic therapy.

This model is based upon the infection induced, by means of oral administration of CD, in mice subjected to wide-spectrum antibiotic treatment.

The aim of the study was to verify the effect of the supplementation with *L. reuteri* LMG P-27481 in preventing/treating the colonization due to challenge with CD after alteration of the intestinal microbial flora due to antibiotic therapy. In this experimental model 6-week-old mice C57BI/6 were treated for 10 days with a wide-spectrum antibiotic (cefoperazone 0.5 mg/ml), known to cause a significant dysbiosis. A group of animals did not receive the antibiotic treatment and it was considered as check (Group 1).

After 48 hours from the end of the antibiotic therapy the mice received a challenge with a suspension of CD strain VPI 10463 (10⁵ CFU).

The treatment with the probiotic *L. reuteri* LMG P-27481 (10⁹ CFU/die) by intragastric route was performed for 8 days after the end of the antibiotic therapy + 2 days preceding the conclusion of the antibiotic administration (Group 3). Another trial group received the probiotic in the same daily doses of group 3, but starting from 24 hours after the challenge with CD (Group 4). The animals of Group 2 received only antibiotic and CD, without probiotic.

The animals were sacrificed 5 days after the challenge with CD and the following determinations were performed:
a) change in body weight during the trial (T0 = first dose antibiotic versus sacrifice);
b) colonization of CD in the caecum by means of real-time PCR analysis, culture by means of sowing in selective medium to quantify the charge of CD in the caecum and research/titration of toxins of CD in the caecal lumen with cytotoxicity assay on monolayer of fibroblasts;
c) entity of inflammation in the mucous membrane of caecum/colon with MPO dosage and histological analysis of sections of caecum and colon.

### Body weight

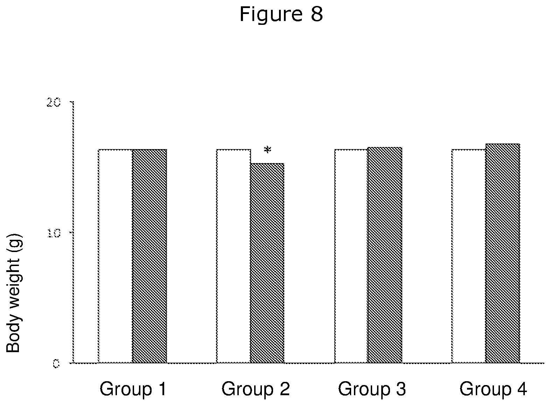

The checking animals not treated with antibiotic showed a relatively stable body weight during the trial, whereas the animals treated with antibiotic and subsequently infected with CD showed a significant reduction in the body weight in 5 days after CD infection. The administration of *L. reuteri* LMG P-27481, both before and after the challenge with CD, abolished completely the weight loss due to CD infection (Figure 8).

### Content of caecum

### Cytotoxicity

After the mice sacrifice, the caecum content was collected, centrifuged and in the obtained supernatant the cytotoxicity, due to CD toxins on a monolayer of Vero cells, was determined.

The faecal content of the checking mice (Group 1) did not show any cytotoxic effect on the Vero cells, contrary to what happened for the mice treated with antibiotic and subsequently infected with CD wherein a cytotoxicity in the order of 80% was noted. The treatment of animals with *L. reuteri* LMG P-27481, both before and after infection with CD, reduced drastically the cytotoxicity induced by CD.

### Toxin A and Toxin B

In order to confirm the presence of the toxins due to CD in the intestine the toxins A and B in the caecal content were determined by means of immuno-enzyme test.

The animals of group 1 (check) obviously were negative, whereas after the treatment with antibiotic and CD (group 2) were strongly positive (++++). The preventive treatment with *L. reuteri* LMG P-27481 (group 3) reduced positivity (++/+++) whereas the treatment subsequent to the CD infection (group 4) determined a complete negativity.

### Presence of C. difficile

The presence of CD in the intestine, was further determined by means of PCR performed on the total DNA extracted from faeces.

The obtained results are in line with what found in the preceding tests. In fact, as expected, the faecal DNA of group 1 was negative for CD, group 2 was strongly positive, groups 3 and 4 resulted slightly positive and negative (compared to the check), respectively.

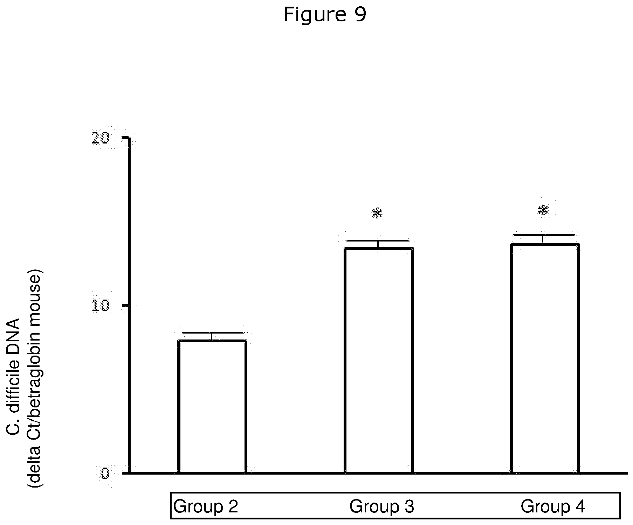

The half-quantitative data obtained with PCR were confirmed by means of real-time PCR test. Figure 9 shows how the mice belonging to group 2, subjected only to the antibiotic therapy and challenge with CD, revealed to be strongly colonized by CD, by showing very low delta-Ct values. Such values instead resulted to be higher in the mice of groups 3 and 4 by demonstrating a very reduced CD colonization.

### Test in Culture

The PCR data were confirmed by plating an aliquot of the caecum content in plates of agar blood and by proceeding with the identification of the colonies by means of MALDI-TOF analysis. Even this test supplied results which can be overlapped to those obtained in the previously described tests. In fact, it was found that the treatment with the probiotic reduces the CS colonies, by showing a better effect for *L. reuteri* LMG P-27481 administered after the challenge.

### Myeloperoxidase (MPO)

The level of infiltration by neutrophils in the mucous membrane was evaluated by measuring the level of MPO activity.

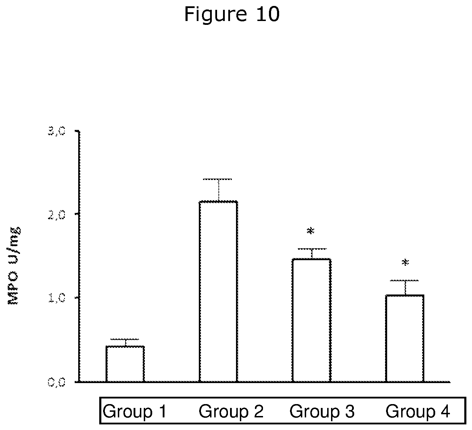

The obtained data are shown in Figure 10, which shows a significant increase in MPO activity in the mucous membrane of the mice belonging to group 2 (antibiotic therapy + challenge CD) with respect to the checking mice, by showing the presence of an acute inflammation. The administration of *L. reuteri* LMG P-27481 starting from a time phase preceding the challenge with CD (group 3) reduced MPO activity by 25% whereas the administration after the challenge (group 4) determined a reduction in MPO activity higher than 50% with respect to group 2.

The entire *in vitro* analytical surveys performed on the strain *L. reuteri* LMG P-27481 showed for this microorganism a strong probiotic activity and allowed to collect elements in favour of the use of strain in diarrhoeas of various aetiology. Such evaluation is based upon the capability of the strain *L. reuteri* LMG P-27481 to use lactose (inner data), thus ideally being capable of contributing to relieve the symptoms of maldigestion in intolerant subjects, and to contrast the growth of *Clostridium difficile,* by reducing by about 1 logarithm in the 24 hours the pathogen development, differently from the checking strains which revealed to be substantially ineffective. The strain *L*. *reuteri* LMG P-27481 further reduces the growth of Gram-negative intestinal pathogens *E. coli* and *Salmonella,* differently to what performed by *Lactobacillus rhamnosus* ATCC 53103 which does not show any inhibiting activity against these pathogens, and *by Lactobacillus reuteri* DSM 17938 which shows reduced activity or similar to that of L. *reuteri* LMG P-27481 in relation to the different tested microorganisms.

A separate discussion should be made for *C*. *difficile* therefor only the strain *L. reuteri* LMG P-27481 showed to be capable of reducing significantly the growth thereof.

The strain *L. reuteri* LMG P-27481 further showed the capability of adhering to the intestinal cells in culture at equal or higher levels than other tested strains and to induce actively the anti-inflammatory stimulation in the dendritic cells in culture, particularly when considered in form of live and vital cells. Such capability reveals to be of particular interest exactly with reference to *C*. *difficile,* known for the capability of inducing a massive inflammation stimulus with significant increase in IL-8 expression. The data obtained in the murine models of *C*. *difficile* infection, both spontaneous infection induced by antibiotic treatment and infection due to challenge with CD, show that the administration of *L. reuteri* LMG P-27481 determines a significant effectiveness contribution in the reduction of the seriousness of the effects associated to the infection.

All experimental data obtained on the strain *L. reuteri* LMG P-27481 demonstrate a considerable effectiveness of this microorganism in the treatment of the intestinal affections due to various origin. In particular a strong reduction in the inflammatory states induced by several stimuli appeared, thus by making this strain a useful instrument in treating acute diarrhoae.

### Bibliography

1) Harrison, Principi di medicina interna, 168 ed., Milano, McGraw-Hill, 2005
2) Guarino A, et al. J Pediatr Gastroenterol Nutr. 2014 Jul;59(1):132-52
3) Bozzani A. I probiotici neldiarrhoea acuta. Rivista della SIMG, n.5 ottobre 2010
4) Lo Giudice M., Bottaro G., Santucci A., Montanari G. Manuale di gastroenterologia pediatrica. Springer-Verlag Ed. 2007: 220
5) Probiotici: attualità e nuove prospettive terapeutiche in pediatria. Adis Int Ltd Ed. 2003: 1-10
6) Boirivant M., Strober W. The mechanism of action of probiotics. Curr Opin Gastroenterol 2007; 23(6): 679-692
7) Matsuzaki T., Takagi A., Ikemura H. et al. Intestinal microflora: probiotics and autoimmunity. J Nutr 2007; 137: 798S-802S
8) Rolfe R.D. The role of probiotic cultures in the control of gastrointestinal health. J Nutr 2000; 130: 396S-402S
9) Elmer GW. Probiotic "living drugs". Am J Health-Syst Pharm 2001; 58(12): 1101-1109
10) Marteau P.R., deVrese M., Cellier C.J. et al. Protection from gastrointestinal disease with the use of probiotics. Am J Clin Nutr 2001; 73(2): 430S-436S
11) Allen S.J. Probiotics for treating infectious diarrhoea. Cochrane Rev Abstract. 2007
12) Bausano G. I batteri amici. II pensiero Scientifico Ed. 2000: 24-25
13) Bindels J, Goedhart AC. Use of probiotics and fibers for diarrhoea. US20110014167A1
14) Casas IA, Mollstam B. Treatment of diarrhoea. US005837238A
15) Chang YH, et al. Novel lactobacillus reuteri useful as probiotics. WO2002070670A1
16) Mogna G. Strains of lactic acid bacteria and/or bifidobacteria inhibiting/reducing the growth of different biotypes of e. coli and different biotypes of clostridia. EP2753687A1
17) EFSA Panel on Additives and Products or Substances used in Animal Feed (FEEDAP); Guidance on the assessment of bacterial susceptibility to antimicrobials of human and veterinary importance. EFSA Journal 2012;10(6): 2740
18) Anderson D, McKay LL. Simple and rapid method for isolating large plasmid DNA from lactic streptococci. Appl Environ Microbiol 1983;46: 549-552
19) Charteris WP, et al. Development and application of an in vitro methodology to determine the transit tolerance of potentially probiotic Lactobacillus and Bifidobacterium species in the upper human gastrointestinal tract. J Appl Microbiol 1998;84: 759-768
20) Gilliland SE, et al. Importance of bile tolerance of Lactobacillus acidophilus used as a dietary adjunct. J Dairy Sci 1984;67(12): 3045-51
21) Yap PS, Gilliland SE. Comparison of newly isolated strains of Lactobacillus delbrueckii subsp. Lactis for hydrogen peroxide production at 5 degrees C. J Dairy Sci 2000;83(4) :628-32
22) Mileti E, et al. Comparison of the immunomodulatory properties of three probiotic strains of Lactobacilli using complex culture systems: prediction for in vivo efficacy. PLoS One 2009;16;4(9): e7056
23) Pietilä TE, et al. Activation, cytokine production, and intracellular survival of bacteria in Salmonella-infected human monocyte-derived macrophages and dendritic cells. J Leukoc Biol 2005;78(4): 909-20

## Claims

1. A strain of *Lactobacillus reuteri* No. LMG P-27481 deposited with the center of collection of microorganisms BCCM/LMG Bacteria Collection of the Gent University (Belgium).

2. The strain according to claim 1 for use in the treatment of diarrhoeal infections, wherein said diarrhoeal infections are induced by *Clostridium difficile.*

3. Pharmaceutical compositions comprising the strain as claimed in at least one of claims 1 to 2 and pharmaceutically tolerable additives.

4. The compositions according to claim 3 for use in the treatment of diarrhoeal infections, wherein said diarrhoeal infections are induced by *Clostridium difficile.*

5. Medical devices comprising the strain as claimed in at least one of claims 1 to 2 and additives used in the field.

6. Medical devices according to claim 5 for the treatment of diarrhoeal infections wherein said diarrhoeal infections are induced by *Clostridium difficile.*

7. Food supplements useful in the treatment of diarrhoeal infections comprising the strain as claimed in at least one of claims 1 to 2 and additives usually used in the nutraceutical field, wherein said diarrhoeal infections are induced by *Clostridium difficile.*

## Patentansprüche

1. Stamm von *Lactobacillus reuteri* Nr. LMG P-27481, hinterlegt bei dem Zentrum für die Sammlung von Mikroorganismen BCCM/LMG Bakteriensammlung der Universität Gent (Belgien).

2. Stamm nach Anspruch 1 zur Verwendung bei der Behandlung von Durchfallinfektionen, wobei die Durchfallinfektionen durch *Clostridium difficile* hervorgerufen werden.

3. Pharmazeutische Zusammensetzungen, umfassend den Stamm nach mindestens einem der Ansprüche 1 bis 2 und pharmazeutisch verträgliche Zusatzstoffe.

4. Zusammensetzungen nach Anspruch 3 zur Verwendung bei der Behandlung von Durchfallinfektionen, wobei die Durchfallinfektionen durch *Clostridium difficile* hervorgerufen werden.

5. Medizinische Vorrichtungen, umfassend den Stamm nach mindestens einem der Ansprüche 1 bis 2 und in diesem Bereich verwendete Zusatzstoffe.

6. Medizinische Vorrichtungen nach Anspruch 5 für die Behandlung von Durchfallinfektionen, wobei die Durchfallinfektionen durch *Clostridium difficile* hervorgerufen werden.

7. Nahrungsergänzungsmittel, die bei der Behandlung von Durchfallinfektionen verwendbar sind, umfassend den Stamm nach mindestens einem der Ansprüche 1 bis 2 und üblicherweise in dem Nutraceuticalbereich verwendete Zusatzstoffe, wobei die Durchfallinfektionen durch *Clostridium difficile* hervorgerufen werden.

## Revendications

1. Souche de *Lactobacillus reuteri* No. LMG P-27481 déposée au centre de collection de micro-organismes BCCM/LMG Bacteria Collection de l'Université de Gand (Belgique).

2. Souche selon la revendication 1 pour utilisation dans le traitement des infections diarrhéiques, dans laquelle lesdites infections diarrhéiques sont induites par *Clostridium difficile.*

3. Compositions pharmaceutiques comprenant la souche selon au moins l'une des revendications 1 à 2 et additifs pharmaceutiquement tolérables.

4. Compositions selon la revendication 3 pour utilisation dans le traitement des infections diarrhéiques, dans lesquelles lesdites infections diarrhéiques sont induites par *Clostridium difficile.*

5. Dispositifs médicaux comprenant la souche selon au moins une des revendications 1 à 2 et additifs utilisés dans le domaine.

6. Dispositifs médicaux selon la revendication 5 pour le traitement d'infections diarrhéiques, dans lesquels lesdites infections diarrhéiques sont induites par *Clostridium difficile.*

7. Compléments alimentaires utiles dans le traitement des infections diarrhéiques comprenant la souche selon au moins l'une des revendications 1 à 2 et des additifs habituellement utilisés dans le domaine nutraceutique, dans lesquels lesdites infections diarrhéiques sont induites par *Clostridium difficile.*
